# EUROPEAN PATENT APPLICATION

(11) **EP 4 042 961 A1**
(43) Date of publication of application: **17.08.2022**
(21) Application number: 20874366.6
(22) Date of filing: 31.07.2020
(51) Int. Cl.: A61B 18/14

(54) **DUAL-ELECTRODE PROBE AND OPERATION METHOD THEREFOR**

(30) Priority: 11.10.2019 CN 201910965089
(71) Applicant: Shenzhen Neumann Technology Co., Ltd., Shenzhen, Guangdong 518000 (CN)
(72) Inventor: LAI, Shen, Shenzhen, Guangdong 518000 (CN)
(74) Representative: Roberts, Peter David
(86) International application number: PCT/CN2020/106405
(87) International publication number: WO 2021/068620

(57) **Abstract**

A dual-electrode probe and an operation method therefor. The dual-electrode probe comprises a first insulating sleeve (100), a first electrode (200) and a second electrode (300), the first insulating sleeve (100) being sleeved outside the first electrode (200), the second electrode (300) being sleeved outside the first insulating sleeve (100), and the first electrode (200) being in sliding fit with the first insulating sleeve (100). As the first electrode (200) is in sliding fit with the first insulating sleeve (100), by sliding the first electrode (200) or the first insulating sleeve (100), an end portion of the first electrode (200) is enabled to extend out of the first insulating sleeve (100), and in this case, the distance by which the end portion of the first electrode (200) extends out of the first insulating sleeve (100) can be adjusted according to the situation, that is, the exposure depth of the first electrode (200) can be adjusted; and as the exposure depth of the first electrode (200) has an effect on the working range and intensity of the dual-electrode probe, the exposure depth of the first electrode (200) can be correspondingly adjusted according to practical situations, thus enabling the dual-electrode probe to better perform an ablation treatment on a predetermined position. Thus, the described dual-electrode probe has an adjustable working range, is easy to operate, and can reduce the operation time.

## Description

### TECHNICAL FIELD

The present disclosure relates to the field of medical instrument, and in particular, to a dual-electrode probe and an operation method therefor.

### BACKGROUND

A steep pulse operation results in apoptosis by delivering low-energy electrical pulses to cells, causing cell membranes to be penetrated and be out of balance. Steep pulse therapy instruments deliver a low-energy direct current (LEDC) with high-voltage to ablate soft tissue. This ablation method is called electroporation method or irreversible electroporation (IRE). A dual-electrode probe is one of the key points of the steep pulse operation. An exposed surface of the dual-electrode probe can produce echoes, which can improve the visibility when placed under ultrasound. An exposure depth of the dual-electrode probe determines a height or depth of ablation. When soft tissue of a tumor lesion is large, the steep pulse operation requires two or more single- or dual-electrode probes to achieve an effect of soft tissue ablation. However, due to differences in different lesions, conventional probes cannot be adjusted according to an actual situation, which may affect a surgical effect.

### SUMMARY

Based on the above, the present disclosure is intended to provide an adjustable dual-electrode probe and an operation method therefor, so as to overcome the defects in the prior art.

The solutions are as follows.

A dual-electrode probe includes a first insulating sleeve, a first electrode and a second electrode. The first insulating sleeve is sleeved outside the first electrode, the second electrode is sleeved outside the first insulating sleeve, and the first electrode is slidably fitted with the first insulating sleeve.

In the dual-electrode probe, as the first electrode is slidably fitted with the first insulating sleeve, by sliding the first electrode or the first insulating sleeve, an end portion of the first electrode is extended out of the first insulating sleeve, and in this case, the distance by which the end portion of the first electrode is extended out of the first insulating sleeve may be adjusted according to the situation, that is, the exposure depth of the first electrode may be adjusted; and as the exposure depth of the first electrode has an effect on the working range and intensity of the dual-electrode probe, the exposure depth of the first electrode may be correspondingly adjusted according to practical situations, thus enabling the dual-electrode probe to better perform an ablation treatment on a predetermined position. Thus, the described dual-electrode probe has an adjustable working range and a good working effect, is easy to operate, and may reduce the operation time.

In an embodiment, the second electrode is slidably fitted with the first insulating sleeve.

In an embodiment, the dual-electrode probe further includes a second insulating sleeve, the second insulating sleeve is sleeved outside the second electrode, and the second insulating sleeve is slidably fitted with the second electrode.

In an embodiment, two end portions of the first electrode are a first end portion and a second end portion respectively, two end portions of the second electrode are a third end portion and a fourth end portion respectively, the second end portion and the fourth end portion are both configured to be electrically connected to an external circuit, and in a use state, a front end of the second insulating sleeve, the third end portion, a front end of the first insulating sleeve and the first end portion are disposed to be extended out sequentially.

In an embodiment, the dual-electrode probe further includes a handle, the handle is provided with an accommodating cavity for accommodating the second insulating sleeve, and the second insulating sleeve is disposed to slidably pass through the handle.

In an embodiment, a rear end of the first insulating sleeve is provided with a first sliding block, the fourth end portion is provided with a second sliding block, and a rear end of the second insulating sleeve is provided with a third sliding block. The first sliding block, the second sliding block and the third sliding block are disposed to pass through the handle and to be slidably fitted with the handle, and the second end portion is fixedly arranged in the accommodating cavity.

In an embodiment, a length of the first end portion extended out of the first insulating sleeve is equal to a length of the third end portion extended out of the second insulating sleeve.

In an embodiment, the dual-electrode probe further includes a first wiring and a second wiring, the first electrode is configured to be electrically connected to the external circuit through the first wiring, the second electrode is configured to be electrically connected to the external circuit through the second wiring, and the first wiring and the second wiring are each provided with a spring-like structure.

In an embodiment, the dual-electrode probe further includes a sliding tube. The sliding tube includes a first connecting portion sleeved outside the second wiring and a second connecting portion sleeved outside the second electrode. The first connecting portion is connected to the second connecting portion, and an inner hole of the first connecting portion is communicated with an inner hole of the second connecting portion.

An operation method for applying the dual-electrode probe as described above includes the following steps:
moving the first electrode or the first insulating sleeve, so that an end portion of the first electrode is extended out of the first insulating sleeve;
inserting the first electrode and the second electrode into predetermined positions; and
energizing the first electrode and the second electrode.

In the operation method, an end portion of the first electrode is extended out of the first insulating sleeve, and then the first electrode and the second electrode are inserted into predetermined positions and energized, so that a path is formed between the first electrode and the second electrode. Since the length of the first electrode exposed from the first insulating sleeve may be controlled by moving the first electrode and/or the first insulating sleeve, the exposure depth of the first electrode may also be adjusted according to practical situations, so that the working effect is better, the operation is simple, and the operation time may be saved.

### BRIEF DESCRIPTION OF THE DRAWINGS

FIG. 1 is a half-section view of a dual-electrode probe according to an embodiment of the present disclosure;
FIG. 2 is a schematic enlarged view of area A in FIG. 1;
FIG. 3 is a side view illustrating an assembled first electrode, first insulating sleeve, second electrode and second insulating sleeve according to an embodiment of the present disclosure;
FIG. 4 is an oblique view of the dual-electrode probe according to an embodiment of the present disclosure;
FIG. 5 is a schematic exploded view of the dual-electrode probe according to an embodiment of the present disclosure;
FIG. 6 is a schematic exploded view illustrating a handle, a first sliding block, a second sliding block and a third sliding block according to an embodiment of the present disclosure;
FIG. 7 is a half-section view of the handle according to an embodiment of the present disclosure;
FIG. 8 is a half-section view of the first sliding block according to an embodiment of the present disclosure;
FIG. 9 is a half-section view of the second sliding block according to an embodiment of the present disclosure;
FIG. 10 is a schematic view illustrating an assembled handle, first sliding block, second sliding block and third sliding block according to another embodiment of the present disclosure;
FIG. 11 is a schematic view illustrating an assembled first sliding block, second sliding block and third sliding block according to another embodiment of the present disclosure; and
FIG. 12 is a schematic exploded view illustrating the handle, the first sliding block, the second sliding block and the third sliding block according to another embodiment of the present disclosure.

### List of Reference Numerals:

100: first insulating sleeve, 110: first sliding block, 111: first sliding chute, 111a: first bayonet, 112: first positioning member, 113: first main body portion, 114: first pressing portion, 115: second accommodating groove, 116: second limiting opening, 117: first button, 118: first sleeve member, 119a: third elastic member, 119b: third clamping block, 200: first electrode, 210: first end portion, 300: second electrode, 310: third end portion, 320: second sliding block, 321: second sliding chute, 321a: second bayonet, 322: second positioning member, 323: second main body portion, 324: second pressing portion, 325: third accommodating groove, 326: third limiting opening, 327: second button, 328: second sleeve member, 329a: first elastic member, 329b: first clamping block, 400: second insulating sleeve, 410: third sliding block, 411: third positioning member, 412: third main body portion, 413: third pressing portion, 414: third button, 415: third sleeve member, 416: second elastic member, 417: second clamping block, 500: handle, 510: third sliding chute, 520: first accommodating groove, 530: first limiting opening, 540: adjustable groove, 541: third bayonet, 610: first wiring, 620: second wiring, 700: sliding tube, 710: first connecting portion, 720: second connecting portion.

### DETAILED DESCRIPTION OF THE EMBODIMENTS

To facilitate understanding of the present disclosure, a more comprehensive description of the present disclosure will be given below with reference to the relevant accompanying drawings. Preferred embodiments of the present disclosure are given in the drawings. However, the present disclosure may be implemented in many different forms and is not limited to the embodiments described herein. Rather, these embodiments are provided to make the contents disclosed in the present disclosure more fully understood.

It is to be noted that when one element is referred to as "fixed to" another element, it may be directly disposed on the another element or an intermediate element may exist. When one element is considered to be "connected to" another element, it may be directly connected to the another element or an intermediate element may co-exist. The terms "vertical", "horizontal", "left", "right" and similar expressions used herein are for illustrative purposes only and are not meant to be the only means of implementation.

Unless defined otherwise, all technical and scientific terms used herein have the same meanings as would generally understood by those skilled in the technical field of the present disclosure. The terms used herein in the specification of the present disclosure are for the purpose of describing specific implementations only, and are not intended to limit the present disclosure. The term "and/or" used herein includes any and all combinations of one or more related listed items.

The "first" and "second" in the present disclosure do not represent a specific quantity or order, but are only used for the distinction of names.

In a specific embodiment, "predetermined positions" are regions where lesions appear, such as tumor lesions.

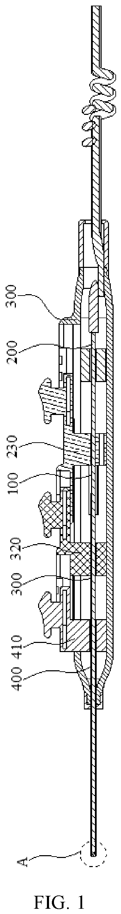

As shown in FIG. 1 to FIG. 3, an embodiment discloses a dual-electrode probe, including a first insulating sleeve 100, a first electrode 200 and a second electrode 300. The first insulating sleeve 100 is sleeved outside the first electrode 200. The second electrode 300 is sleeved outside the first insulating sleeve 100. The first electrode 200 is slidably fitted with the first insulating sleeve 100.

In the dual-electrode probe, as the first electrode 200 is slidably fitted with the first insulating sleeve 100, by sliding the first electrode 200 or the first insulating sleeve 100, an end portion of the first electrode 200 is extended out of the first insulating sleeve 100, and in this case, the distance by which the end portion of the first electrode 200 extended out of the first insulating sleeve 100 may be adjusted according to the situation, that is, the exposure depth of the first electrode 200 may be adjusted; and as the exposure depth of the first electrode 200 has an effect on the working range and intensity of the dual-electrode probe, the exposure depth of the first electrode 200 may be correspondingly adjusted according to practical situations, thus enabling the dual-electrode probe to better perform an ablation treatment on a predetermined position. Thus, the described dual-electrode probe has an adjustable working range and a good working effect, is easy to operate, and may reduce the operation time.

Alternatively, in order to enable the end portion of the first electrode 200 to be extended out of the first insulating sleeve 100, the first electrode 200 may be fixed, and the first insulating sleeve 100 is moved backward; or the first insulating sleeve 100 is fixed, and the first electrode 200 is moved forward; or the first electrode 200 and the first insulating sleeve 100 are moved in a same direction, while displacement of the first electrode 200 is greater than that of the first insulating sleeve 100.

Alternatively, in an axial direction of the first insulating sleeve 100, an end portion of the first insulating sleeve 100 is flush with an end portion of the second electrode 300; or the end portion of the first insulating sleeve 100 is extended out of the second electrode 300. In this case, the first insulating sleeve 100 may have a better insulation effect.

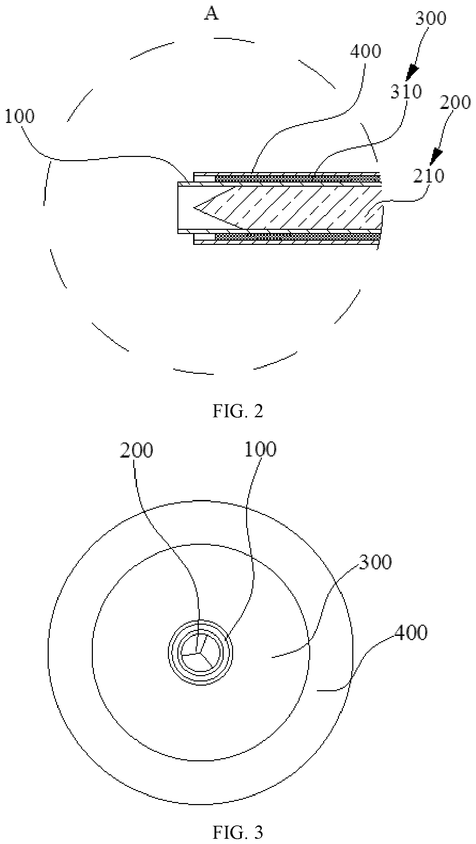

Alternatively, as shown in FIG. 2, an end of the first electrode 200 adapted to be inserted into a predetermined position is of a tip structure. The dual-electrode probe may be easily inserted into the predetermined position.

In an embodiment, the second electrode 300 is slidably fitted with the first insulating sleeve 100. As the second electrode 300 may also be slidably fitted with the first insulating sleeve 100, by adjusting positions between the first electrode 200 and the second electrode 300 on two sides of the first insulating sleeve 100, after the first electrode 200 and the second electrode 300 are inserted into the predetermined positions and energized, the first electrode 200 and the second electrode 300 are electrically connected to form a current, so as to ablate at the predetermined positions. Therefore, a relative position of the first electrode 200 and the second electrode 300 determines a length of a path through which the current flows, and also determines a working range of the dual-electrode probe. That is, by sliding the second electrode 300 relative to the first insulating sleeve 100, the working range of the dual-electrode probe may also be adjusted, so that the dual-electrode probe may be correspondingly adjusted more accurately according to practical situations, thereby improving an operation effect.

In other embodiments, the relative position of the second electrode 300 and the first insulating sleeve 100 may also be fixed. In this case, the exposure depth of the second electrode 300 is constant, and the working range and intensity of the dual-electrode probe may also be adjusted by adjusting only the distance by which the first electrode 200 extended out of the first insulating sleeve 100 to control the exposure depth of the first electrode 200.

In an embodiment, as shown in FIG. 1 to FIG. 3, the dual-electrode probe further includes a second insulating sleeve 400. The second insulating sleeve 400 is sleeved outside the second electrode 300, and the second insulating sleeve 400 is slidably fitted with the second electrode 300. In this case, the exposure depth of the second electrode 300 may be adjusted by controlling the distance by which the second electrode 300 extended out of the second insulating sleeve 400, so that the dual-electrode probe may better adjust the working range and intensity and thus improve the operation effect.

In other embodiments, the second electrode 300 may also be coated with an insulating layer, and the end portion of the second electrode 300 may be conductive. In this case, the exposure depth of the second electrode 300 is constant, but the dual-electrode probe may be adjusted by adjusting the exposure depth of the first electrode 200.

In an embodiment, as shown in FIG. 2, two end portions of the first electrode 200 are a first end portion 210 and a second end portion respectively, two end portions of the second electrode 300 are a third end portion 310 and a fourth end portion respectively, the second end portion and the fourth end portion are both configured to be electrically connected to an external circuit, and in a use state, a front end of the second insulating sleeve 400, the third end portion 310, a front end of the first insulating sleeve 100 and the first end portion 210 are disposed to be extended out sequentially. The front end of the second insulating sleeve 400, the third end portion 310, the front end of the first insulating sleeve 100 and the first end portion 210 are sequentially arranged by movement, so that short circuit may not occur between the first electrode 200 and the second electrode 300. At the same time, the exposure depth of the first electrode 200 is adjusted by the first insulating sleeve 100, and the exposure depth of the second electrode 300 is adjusted by the second insulating sleeve 400, so that the adjustment has higher accuracy, thereby allowing the dual-electrode probe to better process the predetermined position and improving the effect.

Specifically, the "use state" refers to a state of the dual-electrode probe when the dual-electrode probe is to be inserted into the predetermined position after adjustment.

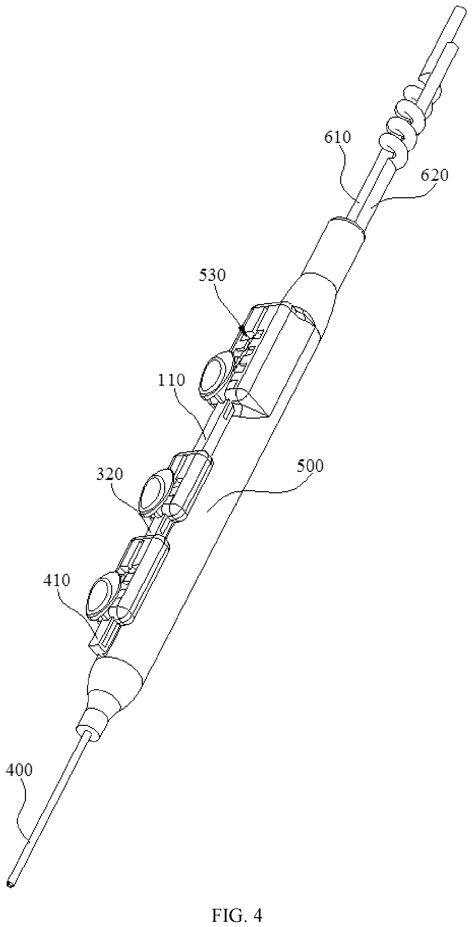

In an embodiment, as shown in FIG. 4, the dual-electrode probe further includes a handle 500, the handle is provided with an accommodating cavity for accommodating the second insulating sleeve, and the second insulating sleeve 400 is disposed to slidably pass through the handle 500. In this case, the second insulating sleeve 400, the second electrode 300, the first insulating sleeve 100 and the first electrode 200 are all partially located in the handle 500. The handle 500 is convenient for a surgeon performing an operation to hold, and the handle 500 may prevent the surgeon from directly touching the electrode, which has good safety.

Alternatively, the handle 500 is tubular, which may reduce the overall size of the dual-electrode probe, and is thus easy to use.

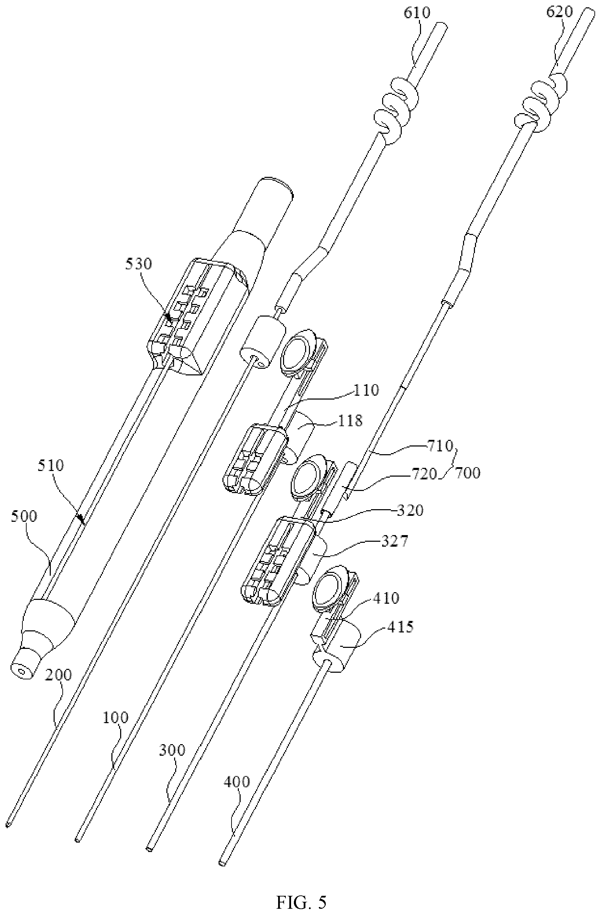

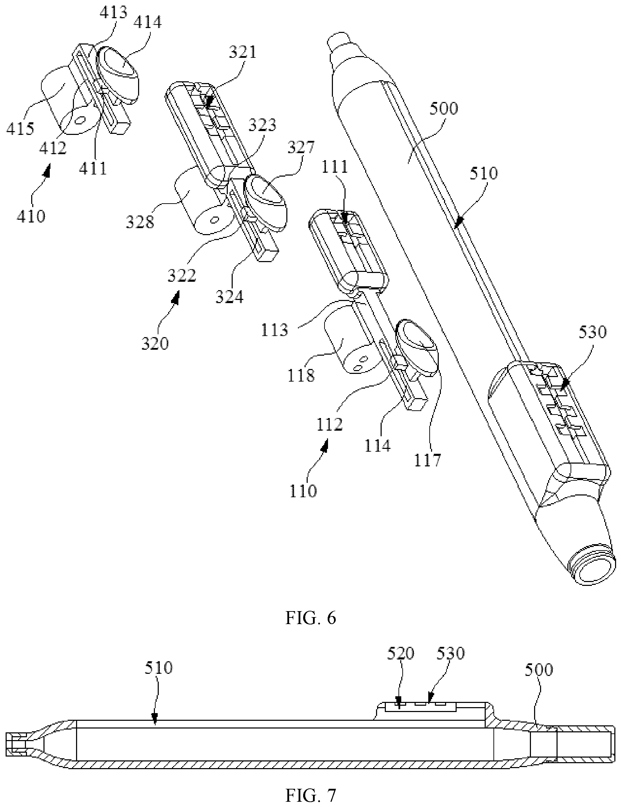

In an embodiment, as shown in FIG. 5 and FIG. 6, a rear end of the first insulating sleeve 100 is provided with a first sliding block 110, the fourth end portion is provided with a second sliding block 320, a rear end of the second insulating sleeve 400 is provided with a third sliding block 410, the first sliding block 110, the second sliding block 320 and the third sliding block 410 are disposed to pass through the handle 500 and be slidably fitted with the handle 500, and the second end portion is fixedly arranged in the accommodating cavity. In this case, exposure depths of the first electrode 200 and the second electrode 300 may be adjusted by sliding the first sliding block 110, the second sliding block 320 and the third sliding block 410 correspondingly. In addition, since the first electrode 200 is not moved in this case, the first insulating sleeve 100 may be moved backward by sliding the third sliding block 410, and thus the first end portion 210 of the first electrode 200 is extended out of the first insulating sleeve 100.

Alternatively, as shown in FIG. 6, the first sliding block 110 is provided with a first sliding chute 111, and the second sliding block 320 is slidably fitted with the first sliding chute 111; the second sliding block 320 is provided with a second sliding chute 321, and the third sliding block 410 is slidably fitted with the second sliding chute 321. In this case, since the third sliding block 410 is limited in the second sliding chute 321 and the second sliding block 320 is limited in the first sliding chute 111, when the first sliding block 110 is moved, the second sliding block 320 and the third sliding block 410 may be driven to move together with the first sliding block 110, so that the first insulating sleeve 100, the second electrode 300 and the second insulating sleeve 400 are moved together, and the exposure depth of the first electrode 200 is adjusted. In this case, an adjustable distance of the second sliding block 320 is a distance by which the second sliding block 320 slides along the first sliding chute 111. Then, the second sliding block 320 is moved to drive the third sliding block 410 to move together, so that the second electrode 300 and the second insulating sleeve 400 are moved together. In this case, an adjustable distance of the third sliding block 410 is a distance by which the third sliding block 410 slides along the second sliding chute 321. Finally, the third sliding block 410 is slid to move the second insulating sleeve 400, and the exposure depth of the second electrode 300 is adjusted. The above structure realizes a sequential adjustment on the exposure depths of the first electrode 200 and the second electrode 300, and ensures the insulation between the first electrode 200 and the second electrode 300. Moreover, the second sliding block 320 and the third sliding block 410 may further adjust their own positions after moving by a certain distance with the first sliding block 110, which reduces the adjustment time and improving the accuracy of adjustment, and is beneficial to reduce the operation time.

In other embodiments, the first sliding block 110, the second sliding block 320 and the third sliding block 410 are slidably arranged at different positions of the handle 500, respectively. In this case, the movements of the first sliding block 110, the second sliding block 320 and the third sliding block 410 are unrelated to each other, and the sliding of the first sliding block 110, the second sliding block 320 and the third sliding block 410 may be adjusted, respectively.

Alternatively, as shown in FIG. 5 to FIG. 7, the handle 500 is provided with a third sliding chute 510 slidably fitted with the first sliding block 110, the second sliding block 320 and the third sliding block 410. A sidewall of the third sliding chute 510 is provided with a first accommodating groove 520. The first sliding block 110 is provided with a first positioning member 112 extending into the first accommodating groove 520. The first accommodating groove 520 is arranged along the axial direction of the first electrode 200. The sidewall of the third sliding chute 510 is further provided with at least two first limiting openings 530 spaced apart from each other. The first limiting openings 530 are sequentially arranged along the axial direction of the first electrode 200. The first limiting openings 530 are located above the first accommodating groove 520 and are communicated with the first accommodating groove 520. The first positioning member 112 may be located in the first accommodating groove 520 or the first limiting openings 530. When the first positioning member 112 is located in the first accommodating groove 520, the first sliding block 110 may be moved along the third sliding chute 510. When the first positioning member 112 enters the first limiting openings 530 from the first accommodating groove 520, the first positioning member 112 are limited by the first limiting openings 530, so that the position of the first sliding block 110 in the third sliding chute 510 is relatively fixed. Therefore, the coordination of the first positioning member 112 with the first accommodating groove 520 or the first limiting openings 530 allows the first sliding block 110 to slide or be fixed, which facilitates the adjustment on the position of the first electrode 200.

Specifically, openings on one side of the first limiting openings 530 are located on an outer surface of the handle 500. In this case, it may be directly observed that the first positioning member 112 is located in different first limiting openings 530 to understand the adjustment on the first electrode 200.

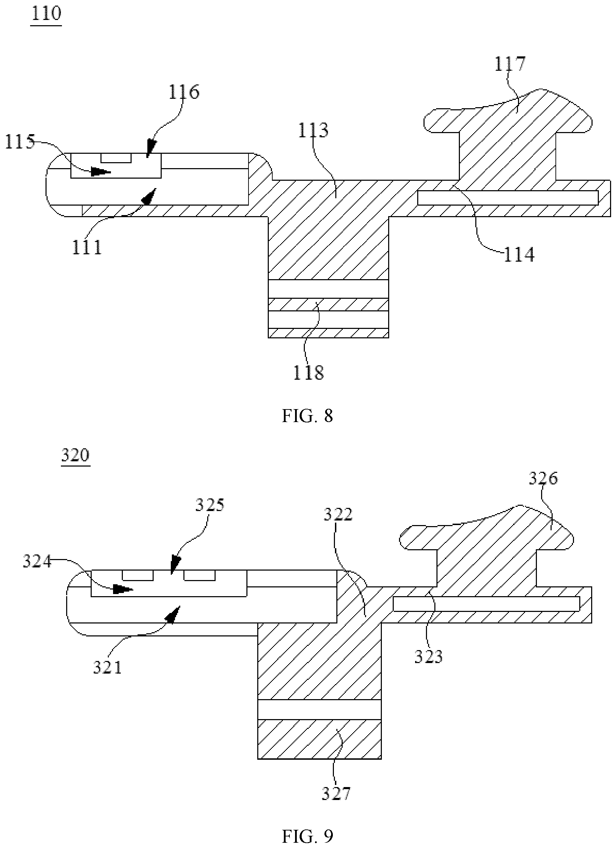

Alternatively, as shown in FIG. 6 and FIG. 8, the first sliding block 110 includes a first main body portion 113 and a first pressing portion 114. The first main body portion 113 is connected to the first pressing portion 114. The first sliding chute 111 is arranged at an end of the first main body portion 113, and the first pressing portion 114 is arranged at the other end of the first main body portion 113. Moreover, a first pressing opening is arranged between the first main body portion 113 and the first pressing portion 114. The first positioning member 112 is connected to the first pressing portion 114. The first pressing portion 114 has a first state and a second state. In the first state, the first positioning member 112 is located in the first limiting openings 530. In the second state, the first pressing portion 114 is pressed and bent toward the first pressing opening, and the first positioning member 112 is located in the first accommodating groove 520. The first positioning member 112 connected to the first pressing portion 114 may be located in the first accommodating groove 520 by pressing the first pressing portion 114. Therefore, the first positioning member 112 may slide along the first accommodating groove 520. That is, the first sliding block 110 may slide along the third sliding chute 510. A distance by which the first electrode 200 extended out is adjusted, and the first pressing portion 114 is released, so that the first positioning member 112 enters the first limiting openings 530. In this case, the first positioning member 112 is limited by the first limiting openings 530, and the position of the first electrode 200 is fixed, so that the first electrode 200 may be conveniently inserted into the predetermined position.

Alternatively, the first sliding chute 111 and the second sliding chute 321 are arranged along the axial direction of the first electrode 200.

Alternatively, as shown in FIG. 6 and FIG. 8, an inner wall of the first sliding chute 111 is provided with a second accommodating groove 115. The second sliding block 320 is provided with a second positioning member 322 extending into the second accommodating groove 115. The second accommodating groove 115 is arranged along the axial direction of the first electrode 200. The sidewall of the first sliding chute 111 is further provided with at least two second limiting openings 116 spaced apart from each other. Different second limiting openings 116 are sequentially arranged along the axial direction of the first electrode 200. The second limiting openings 116 are located above the second accommodating groove 115 and are communicated with the second accommodating groove 115. Specifically, openings on one side of the second limiting openings 116 are located on an outer surface of the first sliding block 110.

Alternatively, as shown in FIG. 6 and FIG. 9, the second sliding block 320 includes a second main body portion 323 and a second pressing portion 324. The second main body portion 323 is connected to the second pressing portion 324. The second sliding chute 321 and the second pressing portion 324 are arranged at two ends of the second main body portion 323, respectively. Moreover, a second pressing opening is arranged between the second main body portion 323 and the second pressing portion 324. The second positioning member 322 is connected to the second pressing portion 324. The second pressing portion 324 has a third state and a fourth state. In the third state, the second positioning member 322 is located in the second limiting openings 116. In the fourth state, the second pressing portion 324 is pressed and bent toward the second pressing opening, and the second positioning member 322 is located in the second accommodating groove 115.

Alternatively, as shown in FIG. 6 and FIG. 9, an inner wall of the second sliding chute 321 is provided with a third accommodating groove 325. The third sliding block 410 is provided with a third positioning member 411 extending into the third accommodating groove 325. The third accommodating groove 325 is arranged along the axial direction of the first electrode 200. The sidewall of the second sliding chute 321 is further provided with at least two third limiting openings 326 spaced apart from each other. Different third limiting openings 326 are sequentially arranged along the axial direction of the first electrode 200. The third limiting openings 326 are located above the third accommodating groove 325 and the third limiting openings 326 are communicated with the third accommodating groove 325. Specifically, openings on one side of the third limiting openings 326 are located on an outer surface of the second sliding block 320.

Alternatively, as shown in FIG. 6, the third sliding block 410 includes a third main body portion 412 and a third pressing portion 413. The third main body portion 412 is connected to the third pressing portion 413. Moreover, a third pressing opening is arranged between the third main body portion 412 and the third pressing portion 413. The third positioning member 411 is connected to the third pressing portion 413. The third pressing portion 413 has a fifth state and a sixth state. In the fifth state, the third positioning member 411 is located in the third limiting openings 326. In the sixth state, the third pressing portion 413 is pressed and bents toward the third pressing opening, and the third positioning member 411 is located in the third accommodating groove 325.

Specifically, a number of the second limiting openings 116 is less than that of the third limiting openings 326, and the number of the second limiting openings 116 is less than that of the first limiting openings 530. Since the sliding of the first sliding block 110 and the third sliding block 410 determines the exposure depths of the first electrode 200 and the second electrode 300, the number of the first limiting openings 530 and the number of the third limiting openings 326 are larger, which may increase an adjustment range and improve the applicability of the dual-electrode probe.

Specifically, as shown in FIG. 6, the first sliding block 110 further includes a first button 117, and the first button 117 is arranged outside the handle 500 and is connected to the first pressing portion 114; the second sliding block 320 further includes a second button 327, and the second button 327 is arranged outside the handle 500 and is connected to the second pressing portion 324; the third sliding block 410 further includes a third button 414, and the third button 414 is arranged outside the handle 500 and is connected to the third pressing portion 413. In this case, it is convenient to press and push the sliding movement.

Alternatively, as shown in FIG. 5 and FIG. 6, the first sliding block 110 includes a first sleeve member 118 sleeved outside the first insulating sleeve 100, the second sliding block 320 includes a second sleeve member 328 sleeved outside the second electrode 300, the third sliding block 410 includes a third sleeve member 415 sleeved outside the second insulating sleeve 400, parts of the first sleeve member 118, the second sleeve member 328 and the third sleeve member 415 located in the handle 500 are matched the accommodating cavity, and the first sleeve member 118, the second sleeve member 328 and the third sleeve member 415 are arranged spaced apart from each other. In this case, the first sliding block 110, the second sliding block 320 and the third sliding block 410 may limit the first insulating sleeve 100, the second electrode 300 and the second insulating sleeve 400, respectively, which may ensure the stability in their radial directions when moving.

Specifically, the first sleeve member 118 is connected to the first main body portion 113, the second sleeve member 328 is connected to the second main body portion 323, and the third sleeve member 415 is connected to the third main body portion 412.

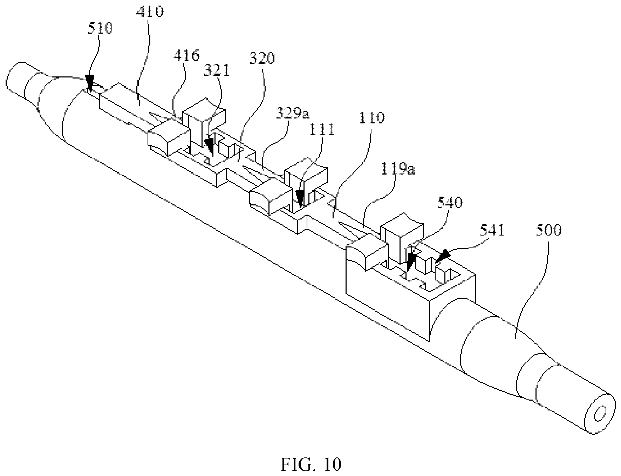

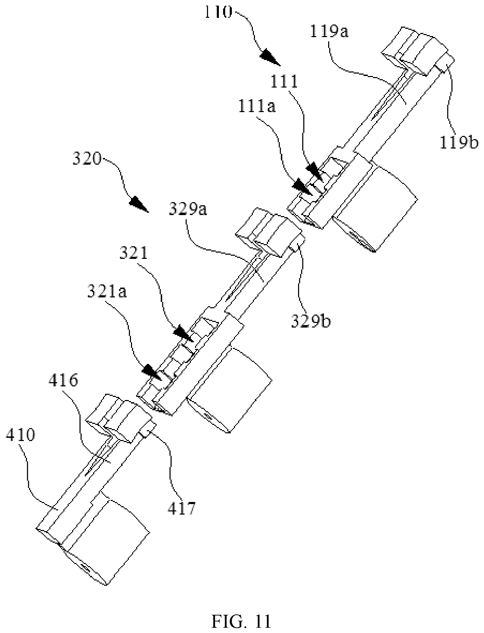

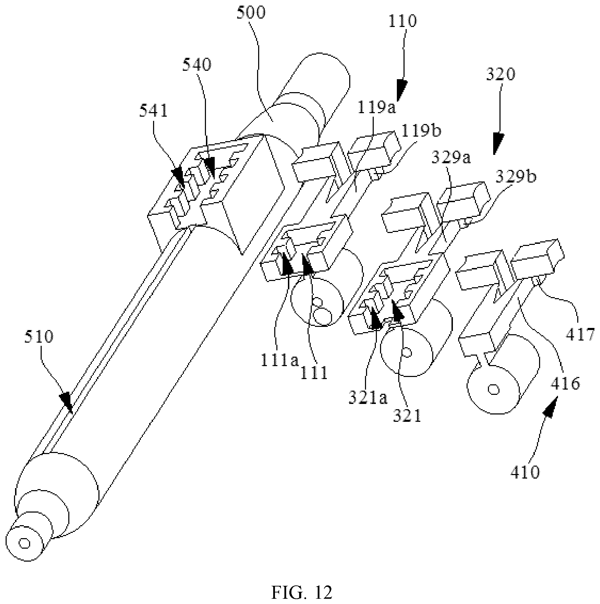

In other embodiments, as shown in FIG. 10 to FIG. 12, the first sliding chute 111 is provided with at least two first bayonets 111a, and the first bayonets 111a are arranged spaced apart from each other along a length direction of the first sliding chute 111. The second sliding block 320 is provided with a first elastic member 329a that may be elastically deformed. The first elastic member 329a has a first normal state and a first bending state. In the first normal state, an end portion of the first elastic member 329a is clamped in the first bayonets 111a. In the first bending state, the first elastic member 329a is detached from the first bayonets 111a and located in the first sliding chute 111. The second sliding chute 321 is provided with at least two second bayonets 321a, and the second bayonets 321a are arranged spaced apart from each other along a length direction of the second sliding chute 321. The third sliding block 410 is provided with a second elastic member 416 that may be elastically deformed. The second elastic member 416 has a second normal state and a second bending state. In the second normal state, an end portion of the second elastic member 416 is clamped in the second bayonets 321a. In the second bending state, the second elastic member 416 is detached from the second bayonets and located in the second sliding chute 321. The handle 500 is provided with an adjustable groove 540. The adjustable groove 540 is arranged along a length direction of the third sliding chute 510. A sidewall of the adjustable groove 540 is provided with at least two adjustable grooves 540. The adjustable grooves 540 are arranged spaced apart from each other along a length direction of the adjustable groove 540. The first sliding block 110 is provided with a third elastic member 119a that may be elastically deformed. The third elastic member 119a has a third normal state and a third bending state. In the third normal state, an end portion of the third elastic member 119a is clamped in the adjustable groove 540. In the third bending state, the third elastic member 119a is detached from third bayonets 541 and located in the third sliding chute 510. In the above structure, alternatively, the previous sliding block may also be moved relative to the following sliding block, and the movement of the following sliding block may simultaneously drive the previous sliding block to move. The sliding block may be moved by a determined distance each time, thereby facilitating the adjustment.

Specifically, as shown in FIG. 10 to FIG. 12, an end portion of the first elastic member 329a is provided with a first clamping block 329b configured to be clamped into the first bayonet 111a; an end portion of the second elastic member 416 is provided with a second clamping block 417 configured to be clamped into the second bayonet 321a; an end portion of the third elastic member 119a is provided with a third clamping block 119b configured to be clamped into the adjustable groove 540.

Specifically, as shown in FIG. 10 to FIG. 12, an end of the first sliding block 110 is provided with a V-shaped notch, and two sides of the V-shaped notch form the third elastic member 119a. An end of the second sliding block 320 is provided with a V-shaped notch, and two sides of the V-shaped notch form the first elastic member 329a. An end of the third sliding block 410 is provided with a V-shaped notch, and two sides of the V-shaped notch form the second elastic member 416.

In an embodiment, a length of the first end portion 210 extended out of the first insulating sleeve 100 is equal to a length of the third end portion 310 extended out of the second insulating sleeve 400. In this case, exposure depths of the first electrode 200 and the second electrode 300 are equal. When a path is formed between the first electrode 200 and the second electrode 300, the current has a stable intensity, which leads to a better use effect.

In an embodiment, as shown in FIG. 4 and FIG. 5, the dual-electrode probe further includes a first wiring 610 and a second wiring 620. The first electrode 200 is configured to be electrically connected to the external circuit through the first wiring 610, and the second electrode 300 is configured to be electrically connected to the external circuit through the second wiring 620. The first wiring 610 and the second wiring 620 are each provided with a spring-like structure. A relative position of the first electrode 200 and the second electrode 300 is required to be adjusted when the exposure depths of the first electrode 200 and the second electrode 300 are adjusted, which may drive the first wiring 610 or the second wiring 620 to move correspondingly. Therefore, the first wiring 610 and the second wiring 620 are each provided with the spring-like structure, so that the first wiring 610 or the second wiring 620 may be stretched or compressed without damages to joints of the first wiring 610 or the second wiring 620 caused by movement.

Alternatively, the first wiring 610 and the second wiring 620 are arranged in parallel. In this case, the overall structure of the dual-electrode probe is compact, which may reduce its radial size and is more convenient to use.

In an embodiment, as shown in FIG. 5, the dual-electrode probe further includes a sliding tube 700, the sliding tube 700 includes a first connecting portion 710 sleeved outside the second wiring 620 and a second connecting portion 720 sleeved outside the second electrode 300, the first connecting portion 710 is connected to the second connecting portion 720, and an inner hole of the first connecting portion 710 is communicated with an inner hole of the second connecting portion 720. The second electrode 300 is sleeved outside the first electrode 200, and a connection between the second electrode 300 and the second wiring 620 may be pulled when the second electrode 300 is moved. Therefore, the connection between the second electrode 300 and the second wiring 620 may be protected by the sliding tube 700.

An embodiment discloses an operation method for applying the dual-electrode probe described above, including the following steps:
moving the first electrode 200 or the first insulating sleeve 100, so that an end portion of the first electrode 200 is extended out of the first insulating sleeve 100;
inserting the first electrode 200 and the second electrode 300 into predetermined positions; and
energizing the first electrode 200 and the second electrode 300.

In the operation method, an end portion of the first electrode 200 is extended out of the first insulating sleeve 100, and then the first electrode 200 and the second electrode 300 are inserted into predetermined positions and energized, a path is formed between the first electrode 200 and the second electrode 300. Since the length of the first electrode 200 exposed from the first insulating sleeve 100 may be controlled by the first electrode 200 or the first insulating sleeve 100, the exposure depth of the first electrode 200 may also be adjusted according to practical situations, so that the working effect is better, the operation is simple, and the operation time may be saved.

Specifically, the step of moving the first electrode 200 or the first insulating sleeve 100, so that an end portion of the first electrode 200 is extended out of the first insulating sleeve 100 may include the following step:
keeping the first electrode 200 stationary, and moving the second electrode 300 and the first insulating sleeve 100 backward, so that an end portion of the first electrode 200 is extended out of the first insulating sleeve 100.

In this case, since the first electrode 200 is kept stationary, the exposure depth of the first electrode 200 may be adjusted only by ensuring that the first insulating sleeve 100 and the second electrode 300 are moved backward, which may reduce the operation time.

In other embodiments, the first insulating sleeve 100 may also be kept stationary, and the first electrode 200 is moved to enable the end portion of the first electrode 200 to be extended out of the first insulating sleeve 100. The above effect may also be achieved.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

The above embodiments only describe several implementations of the present disclosure, and their description is specific and detailed, but may not therefore be understood as a limitation on the patent scope of the disclosure. It should be noted that those of ordinary skill in the art may further make variations and improvements without departing from the conception of the present disclosure, and these all fall within the protection scope of the present disclosure. Therefore, the patent protection scope of the present disclosure should be subject to the appended claims.

## Claims

1. A dual-electrode probe, comprising a first insulating sleeve, a first electrode and a second electrode, the first insulating sleeve being sleeved outside the first electrode, the second electrode being sleeved outside the first insulating sleeve, and the first electrode being slidably fitted with the first insulating sleeve.

2. The dual-electrode probe of claim 1, wherein the second electrode is slidably fitted with the first insulating sleeve.

3. The dual-electrode probe of claim 2, further comprising a second insulating sleeve, the second insulating sleeve being sleeved outside the second electrode, and the second insulating sleeve being slidably fitted with the second electrode.

4. The dual-electrode probe of claim 3, wherein two end portions of the first electrode are a first end portion and a second end portion, respectively, two end portions of the second electrode are a third end portion and a fourth end portion, respectively, the second end portion and the fourth end portion are both configured to be electrically connected to an external circuit, and in a use state, a front end of the second insulating sleeve, the third end portion, a front end of the first insulating sleeve and the first end portion are disposed to be extended out sequentially.

5. The dual-electrode probe of claim 4, further comprising a handle, the handle being provided with an accommodating cavity for accommodating the second insulating sleeve, and the second insulating sleeve is disposed to slidably pass through the handle.

6. The dual-electrode probe of claim 5, wherein a rear end of the first insulating sleeve is provided with a first sliding block, the fourth end portion is provided with a second sliding block, a rear end of the second insulating sleeve is provided with a third sliding block, the first sliding block, the second sliding block and the third sliding block pass through the handle and are disposed to be slidably fitted with the handle, and the second end portion is fixedly arranged in the accommodating cavity.

7. The dual-electrode probe of claim 4, wherein a length of the first end portion extended out of the first insulating sleeve is equal to a length of the third end portion extended out of the second insulating sleeve.

8. The dual-electrode probe of any one of claims 1-7, further comprising a first wiring and a second wiring, the first electrode being configured to be electrically connected to the external circuit through the first wiring, the second electrode being configured to be electrically connected to the external circuit through the second wiring, and the first wiring and the second wiring being each provided with a spring-like structure.

9. The dual-electrode probe of claim 8, further comprising a sliding tube, the sliding tube comprising a first connecting portion sleeved outside the second wiring and a second connecting portion sleeved outside the second electrode, the first connecting portion being connected to the second connecting portion, and an inner hole of the first connecting portion being communicated with an inner hole of the second connecting portion.

10. An operation method for applying the dual-electrode probe of one of claims 1-9, comprising the following steps:
moving the first electrode or the first insulating sleeve, so that an end portion of the first electrode is extended out of the first insulating sleeve;
inserting the first electrode and the second electrode into predetermined positions; and
energizing the first electrode and the second electrode.
